# EUROPEAN PATENT APPLICATION

(11) **EP 1 738 702 A1**
(43) Date of publication of application: **03.01.2007**
(21) Application number: 06253447.4
(22) Date of filing: 30.06.2006
(51) Int. Cl.: A61B 17/66, A61B 17/80, A61C 8/00

(54) **Supra mucosal bone fixation**

(30) Priority: 02.07.2005 GB 0513647
(71) Applicant: Greater Glasgow Health Board, 1345 Govan Road Glasgow, G51 4TF (GB)
(72) Inventor: Campbell, Duncan F. Dept. Oral & Maxillofacial, Glasgow, G51 4TF (GB); Wood Graham A. Dept. Oral & Maxillofacial Surgery, Glasgow, G51 4TF (GB)
(74) Representative: Ede, Eric

(57) **Abstract**

This invention relates to the field of fixation systems for bones. In particular, this invention relates to apparatus suitable for use in the fixation of fractures of, for example, the edentulous mandible. The apparatus includes a plate 200 which is fixed supra-mucosally over the site of the fracture 600 and in the preferred embodiment also includes a removable spacer 100 to prevent unnecessary compression of the mucosa 500 in the surrounding area.

## Description

This invention relates to the field of fixation systems for bones. In particular this invention relates to the field of fixation systems suitable for use in fractures of, for example, the edentulous mandible.

The mandible forms the skeleton of the lower jaw and the inferior part of the face, and is the largest and strongest facial bone. Fractures of the mandible represent common facial fractures, second in frequency only to the nose.

Treatment of a fractured bone typically comprises bringing the broken ends together (reduction) and fixing them firmly together (fixation), such that there is time for the bone ends to heal (immobilisation).

Reduction of fractures involves realignment of the bones concerned into, or close to, their original anatomic and functional relationship; for example the occlusion of the teeth of the upper and lower jaw can provide a guide to the pre traumatic position of the bones.

When teeth are absent, or the teeth do not meet due to most or all being absent, the exact anatomic position is less important than a functionally satisfactory position. A fine adjustment can be adequately addressed by modifications to the patient's dentures. Reduction may be accomplished in an open manner, in which the fractured bone is accessed directly, or in a closed manner in which the fracture is reduced without exposing or further exposing the fractured bones. Temporary holding devices may be utilised to assist in this, i.e. to hold the bones in their correct alignment until a more permanent fixation device is fitted.

These two approaches represent categories of techniques for jaw fractures in general and variations exist for different clinical situations. In addition, there is not always a consensus among clinicians as to the ideal technique for each. Closed reduction is in general preferable for comminuted fractures in which the bone is splintered or crushed, for fractures when the bone has a reduced blood supply, where the patient is not fit for a general anaesthetic or where this might add additional risk to health due to age or existing medical conditions. Typically fractures of the edentulous or atrophic mandible occur in a more elderly patient group, and have a poorer blood supply which is proportionately delivered to the bone more via the bone surface (periosteal blood supply) than from blood vessels within bone, (endosteal blood supply).

In open reduction techniques, once the bones involved have been exposed and positioned correctly, fixation, in which the bones are fixed in this alignment, is effected.

Fixation is most commonly achieved by the use of plates which are positioned in a sub-mucosal, generally subperiosteal location and screwed directly onto the bones, to hold them in a stabilised position whilst healing occurs. The standard length of immobilisation required for bone to bone healing is 4-6 weeks. After this time the plates do not have any useful function but remain *in situ* simply because removal would require a further operation. A disadvantage of this is although the plates and screws have entirely served their function after the healing phase, due to their physical properties they can then adversely effect bone reconstitution by shielding the bone from physiological loading.

Various external fixation (closed reduction) devices are known, such as those which wire dentures in position to create a 'gunning splint' or which fix surgical pins through the facial skin to create a link to an external fixation bar. Whilst these procedures are applicable to the edentulous mandible, they each have risks such as the necessity of a general anaesthetic, nerve damage, availability of dentures (may also be broken) and facial scars.

It is accepted that the edentulous mandible poses a particular challenge to the maxillofacial surgeon due in particular to the thin bone and poor blood supply which makes treatment of these fractures difficult. It is generally agreed that while it represents technical difficulty, rigid fixation with large plates for the treatment of these fractures is currently preferable due to the rigidity of fixation on a relatively weak bone.

In treatment of fractures of the edentulous mandible, internal rigid fixation plates are most commonly used, being placed under the mucosa via intra oral incisions. These plates then hold the bones in a rigid position for and beyond the duration of the fixation period. There remain disadvantages associated with this technique.

In the first instance, the process of inserting the fixation plate under the mucosa involves making an incision in the mucosa and stripping or pulling off of the periosteum to expose adequate bone to apply the fixation plate. This stripping off of the periosteum also takes away from the bone a proportion of its blood supply which is essential for healing and defence against the inevitable contamination of the operative area from oral bacteria. This reduction in blood supply is more significant in the elderly and a good proportion of atrophic mandible fractures occur in this sector of the population. In addition, this procedure necessitates the administration of a general anaesthetic to the patient. As patients presenting with edentulous fractures are largely elderly, anaesthesia poses an increased risk. In addition, incision into the mucosa carries with it the risk of nerve damage such that lip numbness is not uncommon and lip weakness is a possibility.

The elderly edentulous mandible is therefore at greater risk of non-union or infection due to a reduced blood supply. Moreover, the bones of patients with edentulous fractures are often atrophied such that large plates are often advocated to achieve adequate fixation requiring large intra oral incisions and further exacerbating the problem.

A further important disadvantage is that the plates remain *in situ* after healing, which can make denture construction difficult or implant placement near impossible.

The present invention identifies the drawbacks of the conventional techniques and procedures, and proposes a fixation apparatus and method which mitigates one or more of the limitations previously addressed.

The aims and objects of the invention will become apparent from reading the following description.

Generally throughout this description reference to the bone should be taken as the bone including the mucosa unless it is stated otherwise.

According to a first aspect of the invention, there is provided apparatus for the fixation of a fractured bone in a body cavity comprising a plate and a means for holding the plate in position for a period of fixation, where the plate is configured such that in use, it is positioned supra-mucosally, and where the means for holding the plate in position are adapted such that they can be passed through the mucosa into the fractured bone.

According to a second aspect of the invention, the apparatus may be supplied as a kit including a selection of parts for providing an assembly for use in reconstructive repair of fractures of the edentulous mandible, the assembly comprising a plate for the fixation of fractured bone, and fasteners for attachment of the plate to bone, wherein the plate is configured to conform generally to a "U"-shape for supra-alignment' with the edentulous mandible, and the fasteners are configured to penetrate the plate and extend perpendicularly and securably into the edentulous mandible.

Preferably the plate comprises a plurality of apertures through which the means for holding the plate in position can be passed.

These apertures may be provided throughout the length of the plate or in spaced groups to provide a selection of fastening points.

Preferably the means for holding the plate in position comprise a plurality of screw fasteners.

The screws may lockingly engage with the apertures in the plate in order to hold the plate in position.

Preferably,each aperture is adapted to selectively receive a fastener having a head, a shank, and a tip, the tip being threaded to cut into and engage bone, the head being shaped to receive a driving tool and to seat into the aperture, and the shank being of sufficient length whereby the plate is retainable by the fastener in a supra-mucosal position. Thus the choice may be made to select an appropriate position to fasten the plate by selecting amongst a series of apertures provided throughout the length of the plate.

In a preferred form, the apparatus includes a spacer element configured to fit in the assembly between the plate and the edentulous mandible. The spacer may be formed from a flexible material that may be cut using a surgical instrument, e.g. by manipulation of a scalpel. A suitable flexible material for the spacer would be a physiologically benign plastics material or a physiologically tolerable resin, preferably in a foamed form shaped to conform to the plate.

The plate may be formed from stainless steel, titanium, vitallium and the like bio-compatible materials.

The plate may be formed from nylon or similar flexible resin/plastic.

Using nylon or a similar flexible resin or plastic adds bulk for strength and it may offer some benefit as it allows micromovement.

Advantageously the fixation apparatus is configured such that the plate is held next to but substantially over the mucosa. This configuration reduces the risk of damage to the mucosa due to crushing or compression during fitting of the plate.

In addition, and advantageously, as the plate is configured to be held in the oral cavity over the mucosa it is not limited by typical size constraints and thus plate strength can be achieved for a reduced width.

Preferably the plate comprises an anterior face which, in use, is located in the oral cavity nearest to the mucosa, and a posterior face opposite the anterior face.

Preferably the apparatus further comprises a means for delimiting a space between the bone and the plate to prevent damage to the mucosa.

Preferably the means for delimiting a space is a removable spacer adapted to delimit the space between the bone and the plate.

Providing a removable and/or detachable spacer has a number of benefits. Initially, when the spacer is held against the bone it is easier to bring the bone into the correct alignment (although this does not need to be exact for the bone to set). Furthermore, the spacer can act as a pocket into which the bone can be pushed, as well as providing a surface for easy positioning of the plate.

Preferably the removable spacer can be excised from the apparatus after the plate is fixed in position.

Most preferably the removable spacer can be cut away after the plate is in position.

Once the plate is fixed in position over the fractured bone, the removable spacer can be taken away to avoid too much bulky material remaining. Cutting away the spacer, for example by using a scalpel, is relatively straightforward and prevents any damage to surrounding tissue e.g. gums. Once the spacer is removed the plate remains slightly raised above over the bone and mucosa.

An alternative option is that the apparatus can be provided with forceps, said forceps having tips adapted such that the limbs of the forceps grip the plate while the sharp tips engage the bone such that the plate is held a fixed distance over the mucosa without crushing it.

Alternatively, the means for delimiting the space between the bone and the plate is a series of sharp projections from the anterior face of the plate.

Optionally the means for delimiting the space between the bone and the plate is a series of extensions from the posterior face.

Preferably the series of extensions from the posterior face comprises a series of cylindrical extensions at the surface of some or all of the apertures.

Preferably the plate comprises a smooth anterior face.

Most preferably all of the surfaces of the plate are smooth.

The provision of smooth surfaces minimises the damage to surrounding tissues e.g. tongue and gum tissue

Optionally the plurality of apertures are of differing sizes.

Optionally the plurality of apertures are of two different sizes.

Alternatively all of the apertures are of the same size.

Preferably the apertures comprise a channel, which when the plate is positioned over the fractured bone run substantially perpendicular to the fractured bone.

Providing the plate with apertures in the form of channels ensures that when the means for holding the plate in position (which are typically screws) are drilled into place, the channel acts as a guide to ensure that the screws are inserted into the top face of the bone substantially perpendicularly. This has a number of benefits and minimises the risk of accidental nerve damage.

Preferably, the apparatus is provided with a first and second means for holding the plate in position where the first means is operable to hold the plate into position temporarily whilst the second means is a permanent locking means.

Alternatively all of the means for holding the plate in position are permanent locking means.

Optionally the first of the means for holding the plate in position is configured to delimit a space between the bone and the plate to prevent damage to the mucosa.

Preferably the means for holding the plate in position comprises one or more screws adapted to engage the plate.

Optionally the means for holding the plate in position comprises a screw with a head and a shaft portion wherein there is an unthreaded portion below the screw head to limit insertion of the screw into a bone to the lower shaft portion of the screw.

Preferably the plate is a confluent shape.

Optionally the plate is segmental.

Preferably the plate has a planar cross-section.

Optionally the plate can be oval, spherical or half-circle in cross-section.

Preferably the plate is provided with a series of grooves for cooperation with an appropriate alignment device.

According to a further aspect of the invention, there is provided a method for the fixation of fractured bone in a body cavity using the apparatus of the first aspect comprising the steps of:
inserting the plate into the body cavity such that the plate is located supra-mucosally, thereby delimiting the mucosal space to prevent damage to the mucosa,
securing the plate in the supra-mucosal position by engaging the means for holding the plate through the mucosa into the fractured bone in order to hold the plate in position.

More preferably the method comprises the steps of:
inserting the spacer element into the body cavity such that the spacer is located supra-mucosally;
inserting the plate into the body cavity such that the plate is located supra-mucosally above the spacer element;
securing the plate in the supra-mucosal position by engaging the means for holding the plate through the plate, spacer and mucosa into the fractured bone.

Yet more preferably the method comprises the additional step of:
excising the spacer element once the plate has been secured.

A preferred embodiment of the invention will now be described with reference to the accompanying drawings in which:
Figure 1 shows an exploded perspective front view of an apparatus for fixation according to a preferred embodiment of the invention; and
Figure 2 shows a cross section view of an apparatus for fixation affixed to the site of a fracture according to the preferred embodiment of the invention; and
Figure 3 shows a cutaway side view of a locking plate according to an embodiment of the invention affixed to the human mandible at a fracture site; and
Figure 4 shows an alternative embodiment of the invention affixed to the human mandible at a fracture site; and
Figure 5 shows a perspective view of a locking plate held in position by a forceps in accordance with an embodiment of the invention.

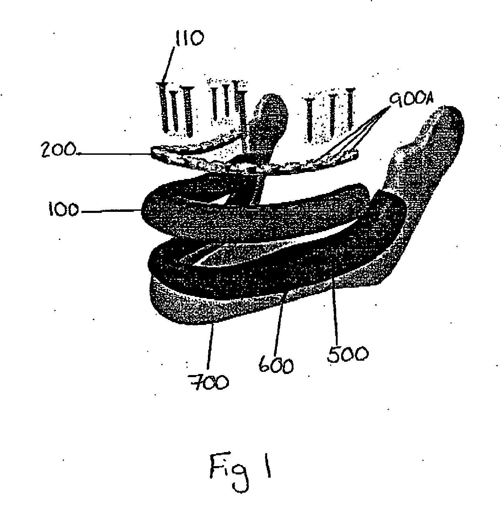

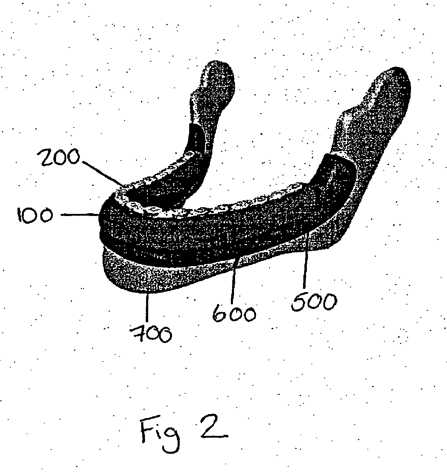

Referring firstly to Figures 1-2, the drawings show an apparatus for fixation of a fractured bone, which, in use, is located substantially within a body cavity. In the preferred embodiment the apparatus for fixation is for use in the mouth in relation to a fracture of the mandible, particularly in the case of an edentulous mandible.

The apparatus for fixation comprises a removable spacer **100** and a plate **200.** The plate **200** is substantially planar in cross section having an anterior face **300** and a posterior face **400** and has a generally smooth surface to resist the accumulation of debris. The plate **200** can be constructed of stainless steel, titanium, vitallium or any suitable bio-compatible material. The spacer 100 is formed of a flexible material, such as plastic or rubber, to allow for convenient manipulation of the spacer **100** within the oral cavity.

Whilst the spacer **100** described is in the form of an insert that can be wholly positioned in the oral cavity and removed once the temporary means for holding the plate in position has been achieved, it will be appreciated that the spacer **100** can take the form of three distinct portions, namely, head, neck and shaft. The shaft portion, which is generally elongated in shape, and head portion, which is substantially planar are joined by the neck portion. The thickness of the head portion is such that it delimits an appropriate amount of space to prevent crushing or compression of the mucosa 500 during the positioning of the plate 200. Furthermore, while the spacer 100 in the depicted and described embodiment is of a flexible construction it will be appreciated that a rigid construction can also be encompassed.

In the preferred embodiment, the apparatus is used by inserting the spacer 100 into the oral cavity against the mucosa 500 at the fracture site 600 before the positioning of the plate 200. The plate 200 is then positioned in a supra-mucosal location against the spacer 100, which serves to delimit the mucosal space and prevent crushing or compression of the mucosa 500 during the procedure. The spacer 100 is held with its anterior surface 300 against the bone 700 spanning the fracture site 600 (over the mucosa 500) to assist in bringing the bone 700 into alignment. The spacer 100 is shaped such that it acts like a pocket into which the bone 700 can be positioned. The posterior surface 400 of the spacer element is provided with an indentation 800 (although in alternative embodiments this can be a smooth surface) into which the plate 200 is placed. The spacer element 100 and plate 200 are both provided with apertures **900a/900b** which can be aligned. The apertures **900a/900b** act as a guide for the screws 110 to ensure that the screws 110 are inserted into the bone 700 at the preferred angle i.e. the screws 110 are inserted through a number of the apertures **900a/900b** substantially perpendicular to the upper surface of the bone **700.** Once in position, the plate 200 is fixed temporarily in place by the insertion of the screws 110 or screw-like awls though the plate **200,** spacer **100** and mucosa **500** into the bone **700.**

Alternative embodiments of the spacer can be envisaged wherein no apertures are provided and the screws are simply screwed through the spacer to form a new aperture during use. Furthermore, the spacer can be provided with head portion and a neck portion formed of a flexible material, such as plastic or rubber, to allow for convenient manipulation of the spacer within the oral cavity.

Once the plate **200** is in position over the bone **700** the spacer **100** can then be removed from the oral cavity. Again, the flexible nature of the spacer **100** will facilitate the easy removal of the device from the oral cavity. Removal of the spacer **100** leaves the plate **200** in position over the bone **700** (and mucosa **500)** but with the plate **200** slightly separated from the mucosa **500** to avoid crushing and discomfort.

In a preferred embodiment the spacer **100** is a relatively soft, flexible material which is then cut free from the screws **110** using a scalpel, without damaging any surrounding tissue, and removed from the oral cavity. Alternative embodiments can be envisaged where the spacer is provided with slits running from the apertures to the outer side of the spacer such that the spacer can be pulled free of the screws without the need for the spacer to be cut whilst *in situ.*

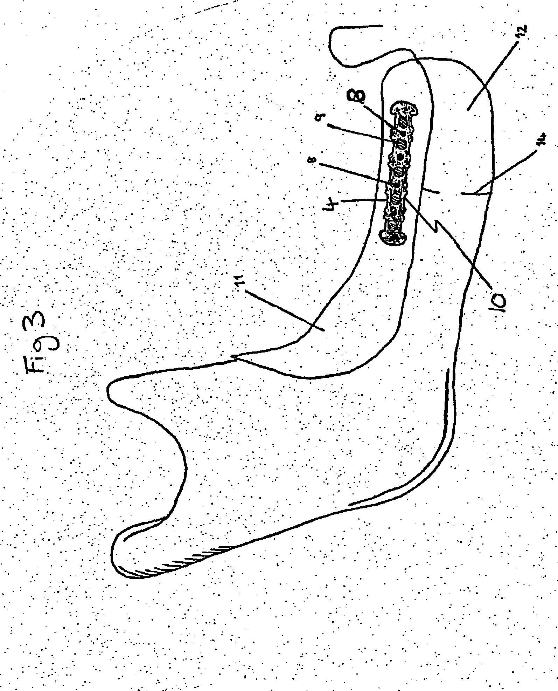

In an alternative embodiment, as shown in figure 3 the plate is provided with a series of grooves **4** along its transverse plane for cooperation with an appropriate alignment device (Not shown).

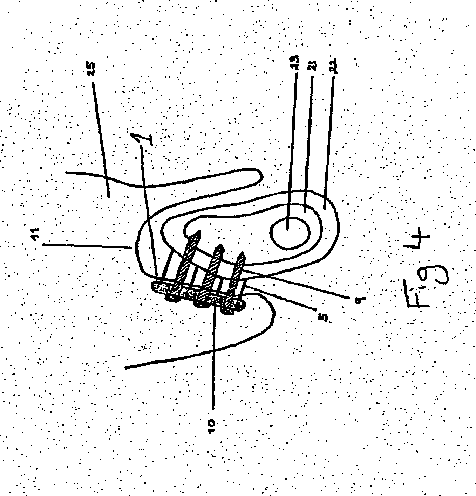

In an alternative embodiment, as shown in figs 3 and 4, where a removable spacer is not used, the anterior face 1 of the plate **10** which, in use faces the mucosa 11, is fitted with a series of sharp projections 5, which serve to keep the plate at a defined distance from the mucosa 11 and thus prevent compression or crushing of the mucosa during positioning of the plate. Thus, the projections serve to create a protective cage around the mucosa which prevents crushing or compression during the fitting of the plate. The plate is provided with a series of apertures **6,7** along its transverse axis **3.** These apertures are of two different sizes **6,7** and are arranged alternately along the length of the plate.

The larger of the two aperture sizes **7,** is configured to receive the locking screws **9** for the plate **10** whilst the smaller aperture size **6** is configured to receive a screw-like awl **8** to temporarily fix the plate into position such that it does not become displaced during the positioning of the plate with the locking screws **9.** The locking screws **9** are operable to lockingly engage with the plate **10** and are configured to conform to the mucosal surface contour.

When using the alternative apparatus which does not include the spacer but instead has projections on the plate, the plate **10** is brought into the supra-mucosal position within the oral cavity. The sharp projections **5** traverse the mucosa **11** and hold the plate **10** in position over the mucosa. The plate **10** is then fixed temporarily in place by the insertion of the screw-like awls **8** though the mucosa **11** into the bone **12.** Before the locking screws **9** are inserted, screw holes are pre-drilled in the bone **12** using a contra-angled handpiece and alignment device. The plate **10** is then lockingly engaged by the fastening of the locking screws **9** to fix the plate into position. Once in place, the supra-mucosal positioning will ensure that any alterations that need to be made to the plate itself, or its positioning, can be effected with minimal difficulty and without the need for incision.

Whilst the mucosal space is delimited by the presence of sharp projections **5** on the anterior face **1** of the plate **10,** it will be appreciated that alternative delimiting means can be employed.

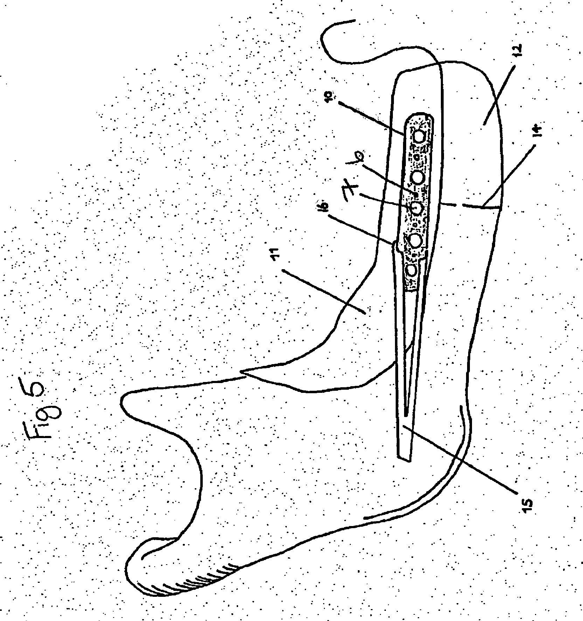

In a yet further alternative embodiment, as shown in figure 5, a modified forceps for use with the fixation system is depicted generally at **15.** The forceps **15** has substantially elongated thin ends **16** which are configured to puncture the mucosa **11** and hold against the bone **12** in order that the forceps can be used to position and hold the plate **10** before it is attached to the bone by the locking screws **9.**

In all embodiments, as the plate **10/200** is positioned within the oral cavity, it is not constrained to a great extent by space, and thus greater plate strength can be achieved for a reduced plate width. It is envisaged that the plate will typically be broader vertically than plates described in the prior art for use in fractures of the edentulous mandible.

It will be evident that various modifications and improvements could be made to the above-described apparatus and methods within the scope of the invention. For example, the above description is written in the context of a fixation plate for application to fractures of the edentulous mandible, however it can be appreciated that the system also has application to the repair of fractures in children with developing dentition, to the repair of fractures to the edentulous mandible, to the repair of fractures of other bones of the body and to bones of non-human animals. As an example, the apparatus could be located in the nasal cavity to achieve fixation of fractures to the nose.

The present invention in one of its aspects provides an improved means for rigid fixation of a fracture of the edentulous mandible which removes the requirement of a general anaesthetic for the procedure. Additionally, the supra-mucosal plate can be removed with minimal difficulty after the requisite healing period, which allows for more straightforward construction of dentures.

Further modifications may be made without departing from the scope of the invention herein intended.

## Claims

1. Apparatus for the fixation of a fractured bone in a body cavity comprising a plate (10; 200) and a means for holding the plate in position for a period of fixation, **characterised in that** the plate is configured such that in use, it is positioned supra-mucosally, and where the means for holding the plate in position are adapted such that they can be passed through the mucosa (11; 500) into the fractured bone (12; 700).

2. Apparatus as claimed in Claim 1, wherein the plate comprises a plurality of apertures (6, 7; 900a, 900b) through which the means for holding the plate in position can be passed.

3. Apparatus as claimed in Claim 2, wherein the means for holding the plate in position lockingly engage with the apertures in the plate in order to hold the plate in position.

4. Apparatus as claimed in any one of the preceding Claims, wherein the means for holding the plate in position comprise a plurality of screws (9, 110).

5. Apparatus as claimed in any one of the previous Claims, wherein the apparatus further comprises a means for delimiting a space between the bone and the plate to prevent damage to the mucosa.

6. Apparatus as claimed in Claim 5, wherein the means for delimiting a space is a removable spacer device (100) adapted to delimit the space between the bone and the plate.

7. Apparatus as claimed in Claim 5 or 6, wherein the removable spacer can be excised from the apparatus after the plate is fixed in position.

8. Apparatus as claimed in any one of the previous Claims, wherein the apparatus is provided with forceps (15), said forceps having tips (16) adapted such that, in use, the limbs of the forceps grip the plate while the sharp tips engage the bone such that the plate is held a fixed distance over the mucosa.

9. Apparatus as claimed in Claim 5, wherein the means for delimiting the space between the bone and the plate is a series of sharp projections (5) from the anterior face (1) of the plate which is located in the oral cavity nearest to the mucosa.

10. Apparatus as claimed in Claim 5, wherein the means for delimiting the space between the bone and the plate is a series of extensions from the posterior face (400) of the plate.

11. Apparatus as claimed in Claim 10, wherein the series of extensions from the posterior face comprises a series of cylindrical extensions at the surface of some or all of the apertures.

12. Apparatus as claimed in any of the previous Claims, wherein the apertures comprise a channel, which when the plate is positioned over the fractured bone run substantially perpendicular to the fractured bone.

13. Apparatus as claimed in any one of the previous Claims, wherein the apparatus is provided with a first and second means for holding the plate in position where the first means (8) is operable to hold the plate into position temporarily whilst the second means (9) is a permanent locking means.

14. Apparatus as claimed in any one of the previous Claims, wherein the means (5) for holding the plate in position is configured to delimit a space between the bone and the plate to prevent damage to the mucosa.

15. Apparatus as claimed in any one of the previous Claims, wherein the means for holding the plate in position comprises a screw with a head and a shaft portion wherein there is an unthreaded portion below the screw head to limit insertion of the screw into a bone to the lower shaft portion of the screw.

16. Apparatus as claimed in any one of the previous Claims, wherein the plate is provided with a series of grooves (4) for cooperation with an appropriate alignment device.

17. An assembly for use in reconstructive repair of fractures of the edentulous mandible comprising a plate (10; 200) for the fixation of fractured bone, and fasteners (9; 110) for attachment of the plate to bone, wherein the plate is configured to conform generally to a "U"-shape for supra-alignment with the edentulous mandible, and the fasteners are configured to penetrate the plate and extend perpendicularly and securably into the edentulous mandible.

18. An assembly as claimed in Claim 17, additionally comprising a spacer element (100) configured to fit between the plate and the edentulous mandible, and formed from a flexible material that may be cut using a surgical instrument.

19. An assembly as claimed in Claim 18, wherein the flexible material may be cut by manipulation of a scalpel.

20. An assembly as claimed in Claim 18 or Claim 19, wherein the flexible material is a physiologically benign foamed form.

21. An assembly as claimed in claim 18 or Claim 19, wherein the flexible material is a shaped plastics material.

22. An assembly as claimed in any one of Claims 17 to 21, wherein the plate comprises a series of apertures (6, 7; 900a, 900b), each aperture being adapted to selectively receive a fastener having a head, a shank, and a tip, the tip being threaded to cut into and engage bone, the head being shaped to receive a driving tool and to seat into the aperture, and the shank being of sufficient length whereby the plate is retainable by the fastener in a supra-mucosal position.

23. An assembly as claimed in any one of Claims 17 to 22, wherein the plate is formed from a material selected from the group consisting of stainless steel, titanium, vitallium and the like bio-compatible materials, and the spacer is formed from a physiologically tolerable flexible resin.
